# EUROPEAN PATENT SPECIFICATION

(11) **EP 1 684 629 B1**
(45) Date of publication and mention of the grant of the patent: **29.04.2009**
(21) Application number: 04808708.4
(22) Date of filing: 11.11.2004
(51) Int. Cl.: A61B 5/0452, A61B 5/04

(54) **METHOD AND DEVICE FOR DETERMINING THE PRESENCE OF AN ISCHEMIC AREA IN THE HEART OF A HUMAN BEING OR AN ANIMAL**
VERFAHREN UND VORRICHTUNG ZUR BESTIMMUNG DES VORLIEGENS EINES ISCHÄMISCHEN BEREICHS IM HERZEN EINES MENSCHEN ODER EINES TIERES
METHODE ET DISPOSITIF PERMETTANT DE DETERMINER LA PRESENCE D'UNE ZONE ISCHEMIQUE DANS LE COEUR D'UN HOMME OU D'UN ANIMAL

(30) Priority: 12.11.2003 NL 1024765
(43) Date of publication of application: 02.08.2006
(73) Proprietor: Consult in Medicine B.V., NL-6211 TG Maastricht (NL)
(72) Inventor: WELLENS, Henrick, Joan, Joost, NL-6211 TG Maastricht (NL); WELLENS, Hein, Maarten, B-4601 Sarolat (BE)
(74) Representative: Valkonet, Rutger
(86) International application number: PCT/NL2004/000792
(87) International publication number: WO 2005/046471

(56) References cited:
- US-A- 5 489 782
- US-A- 5 803 084
- US-A- 5 819 741
- US-A- 2001 025 139

## Description

The invention relates to a device according to claim 1 for carrying out a method for determining the presence of an ischemic area in the heart of a patient having a pain in the chest, comprising the steps of
- A: placing several sense electrodes at different positions on the patient's body;
- B: obtaining electrocardiogram (ECG) signals from the heart via each sense electrode;
- C: determining the presence of the ischemic area in the heart on the basis of the ECG signals obtained in step B.

The term "patient" as used herein is understood to include human beings as well as animals.

In the muscles of the body of a human being or an animal, an electric voltage signal precedes movement of the muscles (contraction and relaxation). Also the heart, which is a hollow muscle that pumps the blood through the body, delivers an electric voltage with every heartbeat. Said electric voltage signal has a characteristic form, the registration thereof is called an electrocardiogram (ECG) in professional circles.

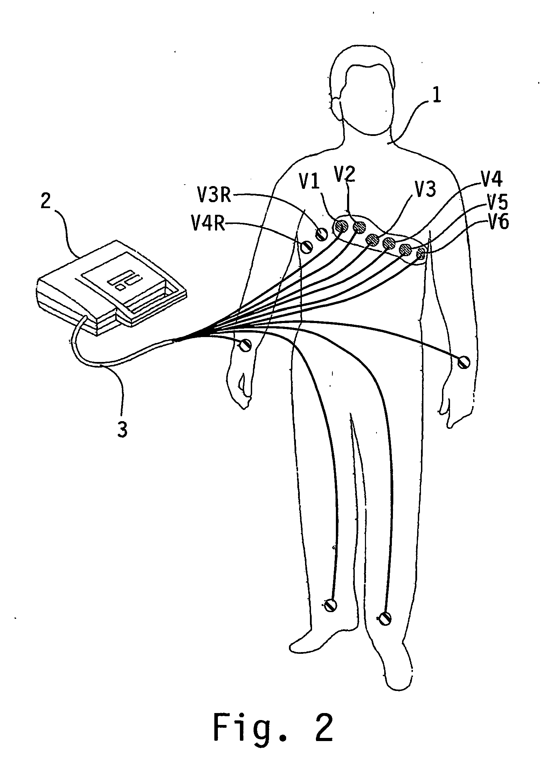

To gain an insight into the plumping action of the heart, including the heart frequency and the electric pulses delivered in the heart and passed through the muscle tissues of the atriums and the ventricles, methods have been developed for measuring the electric voltage signal delivered by the heart. To that end, several sense electrodes are placed on the body (six precordial electrodes on the chest around the heart and four electrodes on the limbs), which electrodes measure the electric voltage signals passing through the heart (see fig. 2).

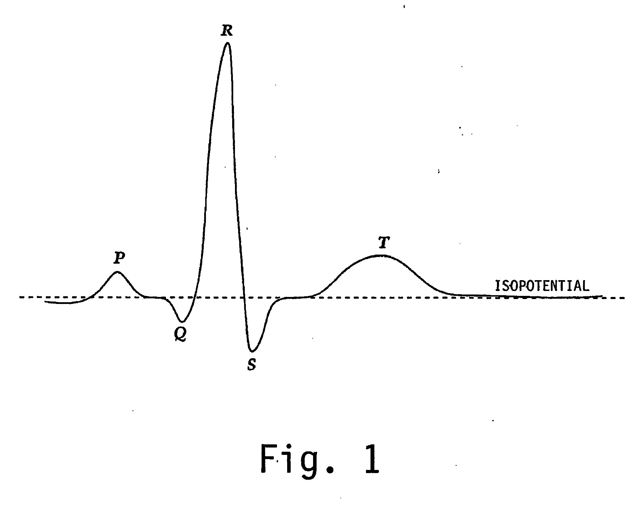

The electrocardiogram thus obtained has a characteristic form for every healthy persons, as is shown in fig. 1. For diagnostic purposes, the ECG signal is divided into several pulses or waveforms over a time interval that corresponds to one heartbeat. The electric activation of the two atriums through the sine node is illustrated by the wave indicated at P in Fig. 1.

In the field of electrocardiography, said activation is called depolarisation. Activation (depolarisation) is followed by the recharging of the heart muscle cells, which is called repolarisation. The repolarisation of the ventricles takes place in the ST segment of the ECG.

With a healthy person, the electrocardiogram as shown in Fig. 1 (P wave, QRS complex and T wave) returns to an "isopotential" level (illustrated in the full line) after every heartbeat.

Aberrations in the ECG (PQRST) in comparison with the ECG of a healthy person may indicate cardiac arrhythmia and damage to the cardiac muscle. Since the first measurement of the ECG took place in the early twentieth century, progress has continuously been made as regards the analysis thereof and as regards the associated diagnosis of potential abnormalities in the cardiac activity.

At present it is possible to determine whether part of the cardiac muscle is not taking part in the pumping action of the heart, or to a lesser extent, for example as a result of a myocardial infarction caused by occlusion of one or more of the coronary arteries, during which the blood circulation in part of the heart was blocked either temporarily or for a prolonged period of time. This can be derived from the ECG signal, because the electric signal passing through the affected heart is different from the ECG signal from a heart that is functioning normally. In professional circles, the apart of the affected cardiac muscle that is not provided with blood is called the "ischemic area".

The determination of the presence of an ischemic area in the heart, and in particular the location and the size thereof, is of vital importance, and the treatment of the patient will be based on said determination. For the treatment it is of paramount importance to know the exact location of the occlusion of the coronary artery, so that said occlusion can be correctly eliminated, and that as quickly as possible.

An example of a methodology or method for determining the presence of an ischemic area in the heart as referred to in the introduction is for example described in US Patent No. zu526. The technique for ECG analysis that is described in said patent publication is only aimed at, and limited to, the determination of the presence of an ischemic area in the heart, however.

A more or less corresponding technique is described in US Patent No. 6,424,860.

A cardiographic display is described in US Patent No. 5,803,084.

So far it has not been possible by means of the currently known ECG analysis methods to obtain additional information with regard to the exact location as well as to the size of the ischemic area in the heart. The availability of this information is of vital importance for the patient in question, however, since the correct treatment scheme and the timely execution thereof can limit the extent of the damage to the cardiac muscle or even prevent such damage altogether.

In particular in the case of a large ischemic area, a quick recovery of the blood supply is essential, which recovery must take place through heart catheterisation, which involves the opening of the occluded coronary artery. The number of hospitals where this treatment can be executed is limited, however. A quick and accurate diagnosis and the right knowledge of the subsequent treatment steps (such as the media to transportation to a specialised cardiac centre) is of paramount importance, therefore.

The object of the invention is to provide a new technique for ECG analysis that meets this purpose.

The method for determining the presence of an ischemic area in the heart comprises the steps of
- D: analysing the various ECG signals obtained in step B; and
- E: determining the location of the occlusion in the coronary artery system of the heart on the basis of the ECG signals as analysed in step D.

More in particular, the method further comprises the step of
- F: determining the size of the ischemic area.

This methodology makes it possible to obtain a direct and accurate assessment of the location and the size of the ischemic area and of the location of the occlusion in the coronary artery system of the heart. This information enables medical personnel (ambulance and hospital personnel or general practitioners) to respond much more quickly and adequately during the acute phase as regards starting a treatment scheme.

To make it possible to determine the location and the size of the ischemic area and the location of the occlusion in the coronary system of the heart, the method further comprises the steps of
- G: determining the isopotential level as well as the deviation of the ST segment from the isopotential level for each ECG signal obtained in step B; and
- H: composing an ST segment deviation vector on the basis of the ST segment deviations obtained in step G.

On the basis of this ST segment deviation vector, the location of the ischemic area in the heart is derived from the direction of the ST segment deviation vector in step I; and the size of the ischemic area as localised in step I is derived from the magnitude of the ST segment deviation vector in step J.

This information enables the medical personnel to draw up an adequate treatment scheme, and to that end the method according to the invention is further characterized by the steps of
- K: projecting the ST segment deviation factor as composed in step H on a schematic representation of the heart; and
- L: displaying the projection as generated in step K to a user.

More specifically, the method according to the invention furthermore comprises the step of
- M: drawing up a scheme of treatment steps on the basis of the location of the occlusion in the coronary artery system of the heart and and the size of the ischemic area as derived in steps E and the location F.

This results in an improved technique for diagnosis and analysis of the electrocardiogram of the heart, and thus in an improved strategy for treating myocardial infarctions. In this way, an adequate, treatment scheme for the patient can be started sooner, which strongly increases the patient's chances of survival.

The device for determining the presence of an ischemic area in a patient's heart in accordance with the invention and claim 1 comprises:
a group of ECG electrodes for measuring ECG signals from the heart of a patient;
ECG data processing means to be connected to the ECG electrodes, which are arranged for receiving the ECG signals and determining the presence of the ischemic area in the heart on the basis of said ECG signals; as well as
display means for displaying ECG data generated by the ECG data processing means to a user.

According to the invention, the device comprises ECG analysis means, which employ a diagnosis and analysis model and further are arranged according to claim 1.

To support the medical 1 personnel in the drawing up of a specific treatment scheme, the display means are arranged for displaying textual information and possibly also for projecting the ST segment deviation vector as composed on a schematic representation of the heart.

An embodiment of a device according to the invention will be explained in more detail hereinafter with reference to a drawing. In the drawing:
Fig. 1 shows an electrocardiogram of a healthy person during one heartbeat;
Fig. 2 shows an embodiment of the device according to the invention;
Fig. 3A is a schematic view of the coronary artery system surrounding a human heart, in which locations where an occlusion may occur are schematically indicated;
Fig. 3B is a schematic view of the coronary artery system surrounding a human heart, in which possible stricture locations are schematically indicated;
Fig. 4 is a flow diagram showing an embodiment of the method carried out by the device according to the invention;
Fig. 5 is a flow diagram showing a second embodiment of the method carried out by the device according to the invention;
Fig. 6 shows a diagnosis and analysis model according to the invention;
Figs. 7A and 7B show a first example A of an ECG diagnosis and analysis according to the invention;
Figs. 8A and 8B show a first example B of an ECG diagnosis and analysis according to the invention;
Figs. 9A and 9B show a third example C of an ECG diagnosis and analysis according to the invention; and
Figs. 10 and 11 are detail views of a second and a third embodiment of a device according to the invention.

Fig. 2 shows an embodiment of a device according to the invention as used with a patient 1. The device 2 according to the invention is provided with a bundle of sense electrodes 3, which are to be applied to the body of the patient 1 in accordance with the configuration or orientation that is known in medical practice. The bundle of sense electrodes 3 consists of six precordial EGG electrodes V1-V6, which are applied to the front side of the patient's thorax, around the heart, as is shown in Fig. 2.

Furthermore, the device 2 according to the invention makes use of four limb sense electrode, which are applied to the left-hand and right-hand arm and the left-hand and righ-hand leg, respectively. By means of said four limb sense electrodes the six extremity derivations (I, II, III, AVF, AVL and AVR) are generated.

By means of said ECG sense electrodes, the electrocardiogram of the human heart is measured from several angles in the form of twelve ECG voltage signals that vary in time, as is shown in Fig. 1. This results in twelve ECG signals.

To achieve a more precise operation of the device according to the invention, use may be made of two additional ECG sense electrodes V3R and V4R, which are placed on the chest, above the right-hand half of the heart, as is shown in Fig. 2. The use of two additional sense electrodes V3R and V4R, respectively, results in a set of fourteen ECG signals, which provide more precise information about the condition and the pumping action of the heart.

The device according to the invention utilises the set of twelve ECG signals or the set of fourteen ECG signals for determining whether an occlusion has occurred in the heart, and in particular in the coronary artery system, as a result of which the heart muscle tissue downstream of the occlusion may become ischemic.

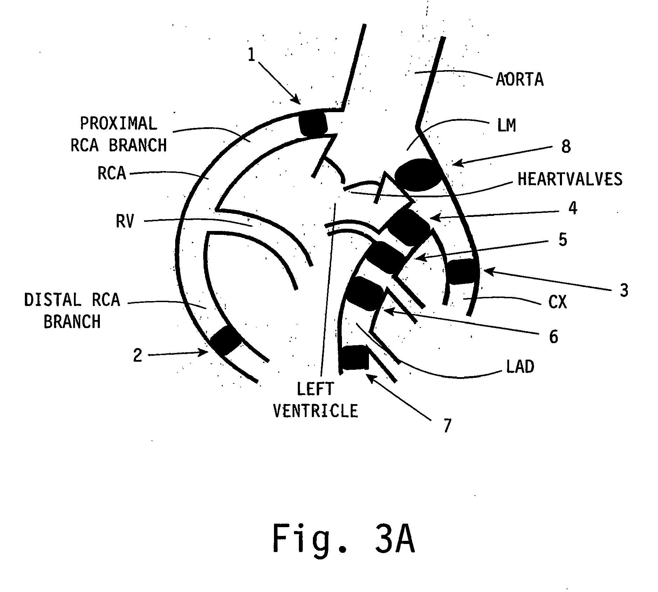

Fig. 3A schematically shows the coronary artery system of a human heart. The coronary artery system is the first main branch of the aorta, which springs from the left-hand ventricle of the human heart, which is shut off by means of heart valves. Oxygen-rich blood is pumped into the aorta from the left-hand ventricle, and part of said aorta branches into the coronary artery system.

The coronary artery system is built up of two coronary arteries. The right-hand coronary artery RCA which, after branching off from the aorta, provides the right-hand side (right-hand ventricle) as well as part of the left-hand ventricle with oxygen-rich blood. The right-hand coronary artery RCA thus provides the entire right-hand side and about 25% of the left-hand side of the heart with blood.

Experts divide the right-hand coronary artery RCA into an upper part, the proximal RCA branch, and a lower part, the distal RCA branch. The proximal part of the RCA extends to a branch that passes over the right-hand ventricle (RV).

The other coronary artery of the coronary artery system is the left-hand main coronary artery LM, which provides the remaining 75% of the left-hand side of the heart with oxygen-rich blood. The left-hand coronary artery LM divides into the left-hand anterior descending coronary artery LAD (50%) and the circumflex coronary artery CX (25%)_{.}

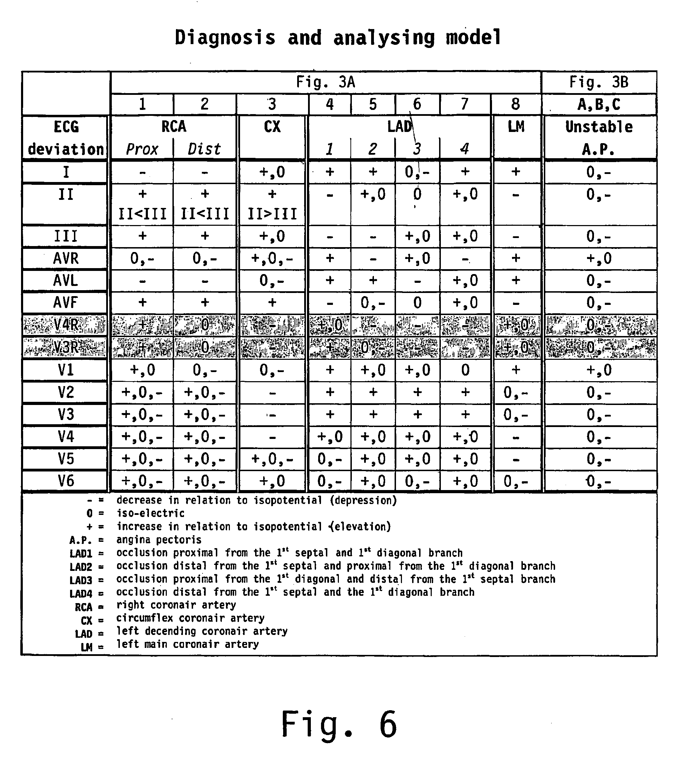

Fig. 3A shows eight locations in the coronary artery system (indicated by numerals 1-8) where an occlusion of the coronary artery in question may occur. The eight locations of possible occlusions can be determined by means of the method and the device according to the invention, which determination will be explained with reference to Figs. 6, 7A-7B, 8A-8B and 9A-9B.

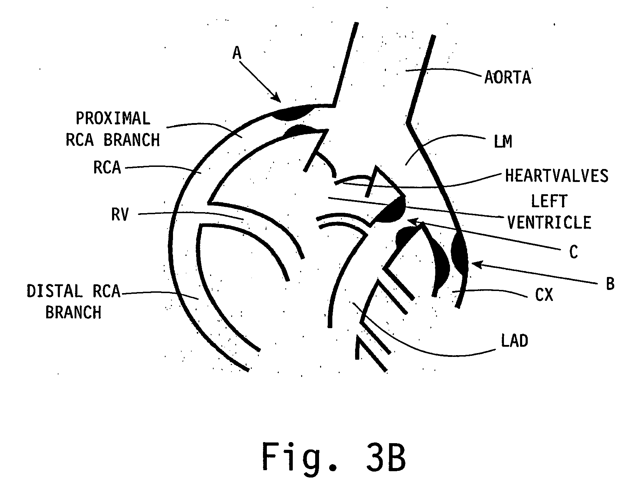

Fig. 3B furthermore shows three situations (indicated by the letters A, B, C) that concern a stricture (angina pectoris) in a specific part of the coronary artery system. The strictures at the locations A, B and C may evolve into an occlusion. The invention and the method carried out by the device according to the invention are explained in more detail with reference to Figs. 6, 7A-B, 8A-8B and 9A-9B on the basis of the latter situation.

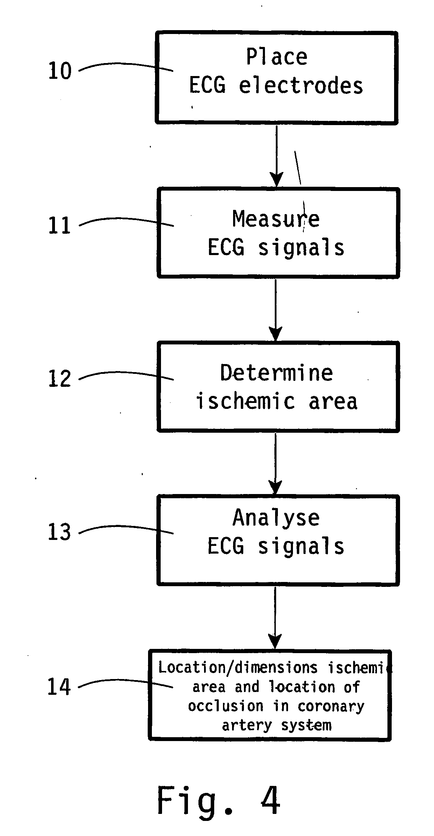

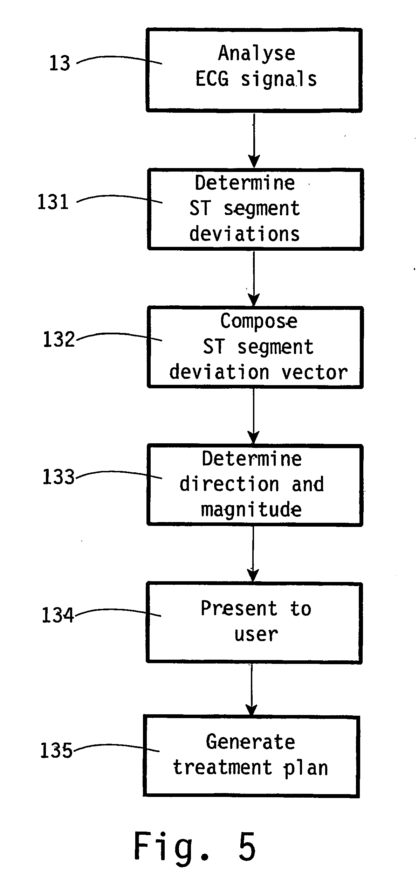

Figs. 4 and 5 show two embodiments of the method carried out by the device according to the invention. The method is best explained with reference to a flow diagram as shown in Figs. 4 and 5. In a first step 10 for carrying out the method, the bundle of ECG electrodes 3 (Fig. 2) is applied to the chest (the six precordial electrodes V1-V6) and the limbs in the known manner.

In the next step 11, the ECG signals from each ECG sense electrode are measured, resulting in a set of nine directly measured electrocardiograms I-III, V1-V6 and three derived electrocardiograms AVF, AFL and AVR.

Subsequently, it is determined in step 12 whether an occlusion or a serious stricture resulting in the formation of an ischemic area in the heart muscle tissue has occurred in the coronary artery system. The determination of the presence of the ischemic area according to step 12 can be carried out in a known manner as described in US Patent No. 6,171,256.

In a subsequent step 13 of the method, the obtained set of ECG signals are further analysed, on the basis of which analysis the exact location and size of the ischemic area and the location of the occlusion in the coronary artery system of the heart are determined.

In Fig. 5, the subsequent step 13 of the method is explained in more detail. In a first special step 131 of the method, the deviations of the ST segment from the isoelectric level (isopotential) in each electrocardiogram are determined in the set of twelve ECG signals (V1-V6, I-III, AVF, AVR, AVL).

In the case of an occlusion or serious stricture in the coronary artery, as a result of which part of the heart muscle tissue has become ischemic, a disturbance will occur in the signal of the electrocardiogram as shown in Fig. 1. In the acute phase, said disturbance will manifest itself in particular in the form of a change in the form of the so-called ST segment that forms the transition between the QRS complex and the T-wave. These changes may concern an elevation or a depression in relation to the isoelectric level ("isopotential").

In the analysis of electrocardiograms, the occurrence of changes (an elevation or a depression) in the ST segment is presently considered to be a first indication that the coronary artery system is not circulating blood optimally through at least part of the heart muscle tissue, which means that said tissue has become ischemic.

The deviations in the level of the ST segment of the various ECG signals from the isoelectric level (isopotential) as determined in step 131 are used for composing a so-called ST segment deviation vector. Said ST segment deviation vector as composed in step 132 comprises a direction and a length (magnitude). The direction of the vector indicates the location of the heart muscle tissue that has become ischemic, and consequently the most probable location of the occlusion or serious stricture (angina pectoris) in the coronary artery system.

The length or magnitude of the ST segment deviation vector, which is determined by finding the aggregate of the absolute values of all determined ST segment deviations of each individual EGG signal, is a measure of the size and the seriousness of the affected ischemic area (step 133).

The electrocardiogram of the heart is an electric voltage signal, wherein the voltage exhibits the PQRST pulse form as shown in Fig. 1 for the duration of a respective heartbeat. The voltage is measured in millivolt (mV). Thus, every deviation in the ST segment in the electrocardiogram from the isoelectric level is measured in millivolt as well. The polarity of the ST segment deviation (characterized in millivolt) is positive (+) in the case of an elevation (increase) and negative (-) in the case of a depression (decrease) in relation to the isoelectric level (isopotential). If the measured ST segment deviation equals 0 millivolt, the isoelectric ST segment (0) is like that of a healthy person.

In step 134 this information is displayed to the user, usually in the form of a textual localisation and size indication, or in the form of a graphic display, in which the composed ST segment deviation vector is projected on a schematic representation of the human heart. Thus, the user (for example ambulance personnel) can directly determine on the basis of this information how serious the patient's infarction is and whether urgent treatment and transportation to a more specialised hospital is considered necessary.

In this decision process, the method can therefore provide a suggestion in step 135 for a treatment scheme to be followed. Said treatment scheme may for example comprise the administration of blood-diluting agents or the immediate transportation to a specialised centre for carrying out a heart catheterisation and opening the occluded coronary artery.

The device according to the invention employs a diagnosis and analysis model for analysing the measured ECG signals in step 13 (and consequently the steps 131-135) in Figs. 4 and 5, an example of which is shown in Fig. 6. In this stable, the first column contains the fourteen relevant ECG signals, being the six precordial ECG signals V1-V6, the optional precordial ECG signals V3R and V4R (illustrated in grey), the three limb signals I, II, III as well as their derived limb signals AVR, AVL and AVF.

The diagnosis-analysis model moreover furthermore discloses a number of columns arranged side by side, which columns each individually describe one of the eight occlusions that are most likely to occur in the coronary artery system. The locations of said eight occlusions are schematically indicated in Fig. 3A, and they are represented accordingly in the diagnosis-analysis model.

The second and the third column, indicated RCA *Prox* and RCA *Dist,* respectively, describe the characteristics of the ST segment deviations for each of the 12 (or 14) ECG signals in the situation in which an infarction/occlusion has occurred in the upper part of the right-hand coronary artery RCA Prox (proximal branch) or in the lower part of the right-hand coronary artery RCA Dist (distal branch) (numerals 1 and 2 in Fig. 3A).

The fourth column CX contains the characteristics of the ST segment deviations corresponding to an infarction/occlusion in the circumflex coronary artery CX (numeral 3 in Fig. 3A), whilst the fifth, sixth, seventh and eight column LAD *1-4* describe the ST segment deviations of four different locations (numerals 4-7 in Fig. 3A) where LAD obstructions (occlusions) may occur in the left-hand anterior descending coronary artery. Refer to Fig. 6 for explanation of LAD *1-4.*

In the ninth column LM, the ST segment deviations associated with an occlusion in the upper part of the left-hand main coronary artery LM (numeral 8 in Fig. 3A) are described. This latter situation can be considered to be a very serious infarction, since as much as 75% of the heart muscle tissue (among which substantially the entire left-hand side of the heart) is deprived of oxygen-rich blood as a result of the occlusion of the left-hand main coronary artery LM. If such an LM occlusion is not immediately removed, this will lead to a life-threatening situation for the patient, as a large portion of the muscle tissue is in danger of becoming permanently ischemic.

In addition to that, the tenth (last) column shows the ST segment changes as encountered with a person having a pain in the chest. In that situation there is no occlusion in one of the coronary arteries yet, to be true, but a serious stricture does occur in several coronary arteries. In professional circles, this situation, which is indicated A-C in Figure 3B, is referred to as "unstable angina pectoris" ("unstable A.P.").

The location of the occlusion or serious stricture in the coronary artery system thus has a determining influence on the size of the heart muscle tissue that has become ischemic, and thus it also has a significant effect on the chances of survival and the heart function in the future. Within the framework of providing direct and adequate care, it is very desirable to gain a timely insight into the location and the size of the occlusion and the ischemic area.

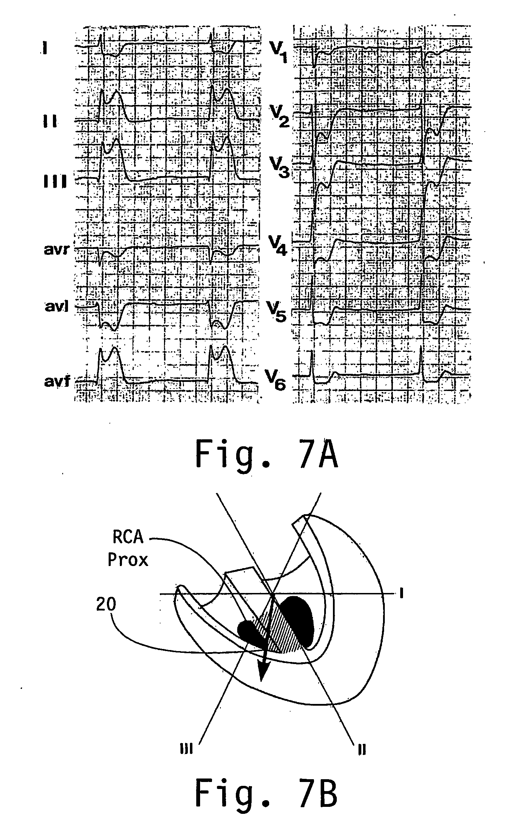

Figs. 7A-7B, 8A-8B and 9A-9B show, in conjunction with Figs. 3 and 6, three situations that disclose the principle of the method and the device according to the invention.

### Example A

This example shows in Fig. 7A a set of twelve ECG signals I-III, AVR, AVF and V1-V6 of a patient who has had an infarction as a result of an occlusion in the coronary artery system. Upon analysis in accordance with step 13 (Figs. 4 and 5) of the method carried out by the device according to the invention it becomes apparent that the ST segment of the ECG signals V2-V6, I, AVR and AVL exhibit a negative deviation ("-" = depression) from the isoelectric level (isopotential).

In addition to that, the ECG signals II, III and AVF exhibit an increased deviation of the ST segment ("+" = elevation) from the isoelectric level. The ECG signal VI exhibits an isoelectric ST segment ("0" = isoelectric). Furthermore it appears that the elevation of the ECG signal II is smaller than the elevation of the ECG signal III.

According to the method, an ST segment deviation vector is composed on the basis of the diagnosis and analysis model of Fig. 6, which vector is indicated at 20 in Fig. 7B. As a result of the elevation of the ECG signals II and III, the ST segment deviation vector 20 is directed toward the position at which the ECG electrodes I and III are placed on the human body. Furthermore, the ST segment deviation vector 20 points away from the location at which the ECG sense electrode I is present, because the ST segment of the ECG signal I exhibits a depression in relation to the isoelectric level.

Using the diagnosis-analysis model as shown in Fig. 6, it can be concluded from the direction and the length/magnitude of the ST segment deviation factor 20 that the occlusion is localised in the proximal branch of the right-hand coronary artery RCA. The location at which the occlusion in the proximal branch of the right-hand coronary artery RCA is present is indicated at 1 in Fig. 3A.

The length of the ST segment deviation vector 20, and consequently the size of the affected ischemic area in the heart, is determined by finding the aggregate of the absolute magnitudes of the various ST segment deviations (elevation, depression, isoelectric) of the ECG signals.

The deviation in each ST segment is determined in millivolt, with the isoelectric level (isopotential) of the electrocardiogram amounting to 0 mVolt. A positive potential difference indicates an "elevation", whilst a negative potential difference indicates a "depression". In this example, the ST segment deviation score is 76 mm.

Using the diagnosis-analysis model according to the invention, it is possible to determine; directly after the analysis of the patient's ECG (the determination of the various ECG signals), the location and the size of the ischemic area and the location of the occlusion in the coronary artery system of the heart and whether direct action is to be taken as regards the treatment of the patient.

As in this Example A the measured ST segment deviations for the various ECG signals correspond most to the values ("+", "0" or "-") of the ST segment deviations that are characteristic of an occlusion in the proximal branch of the right-hand coronary artery RCA *Prox* (see the associated column in Fig. 6 and numeral 1 in Fig. 3A), the fact that the patient in question has such an occlusion can be directly diagnosed by means of the method carried out by the device according to the invention. In this example, the ST segment deviation score is 76 mm.

### Example B

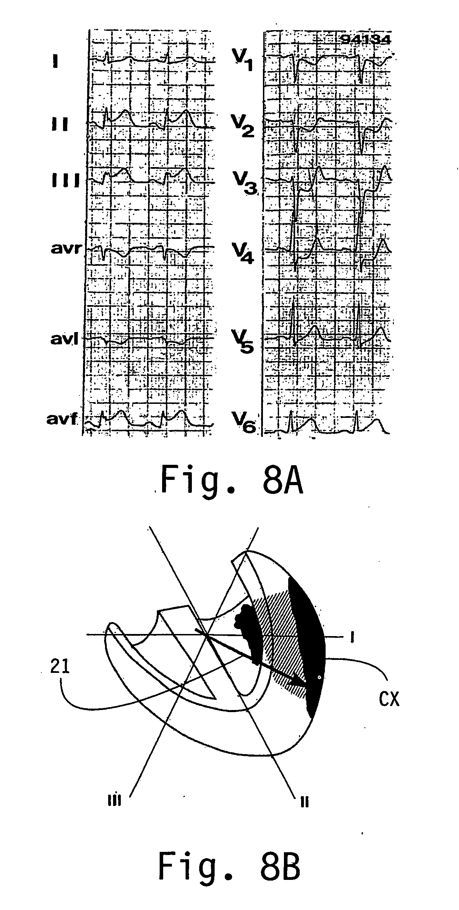

The second example B of the operation of the device according to the invention is shown in Figs. 8A and 8B in conjunction with Fig. 3A and Fig. 6.

Of the twelve ECG signals that are shown in fig. 8A, the ECG signals II, III, AVF, V5 and V6 exhibit an elevation of the ST segment ("+") in comparison with the isoelectric level. A depression of the ST segment ("-") is measured in the ECG signals AVR, AVL, V2, V3 and V4, whilst the ST segments in the ECG signals I, AVL and V1 are isoelectric ("0"). Furthermore, the elevation of the ECG signal II is greater than the elevation of the ECG signal III.

The determination of the twelve ST segment deviations results in a composed ST segment deviation vector 21 in Fig. 8B, which vector 21 is directed towards the circumflex coronary artery CX. In Fig. 3A, the location of the occlusion in the circumflex coronary artery CX is indicated at 3. Furthermore, the polarities of the determined ST segment deviations of the electrocardiograms that are shown in fig. 8A correspond to the ST segment deviations that describe an occlusion in the circumflex coronary artery CX. Refer to the column CX of the diagnosis and analysis model of Fig. 6.

In this example B, the length/magnitude of the ST segment deviation vector 21, and consequently the size of the affected ischemic area in the heart, is determined by the aggregate of the absolute magnitudes of the various ST segment deviations (elevation, depression, isoelectric) of the ECG signals as shown in Fig. 8A.

### Example C

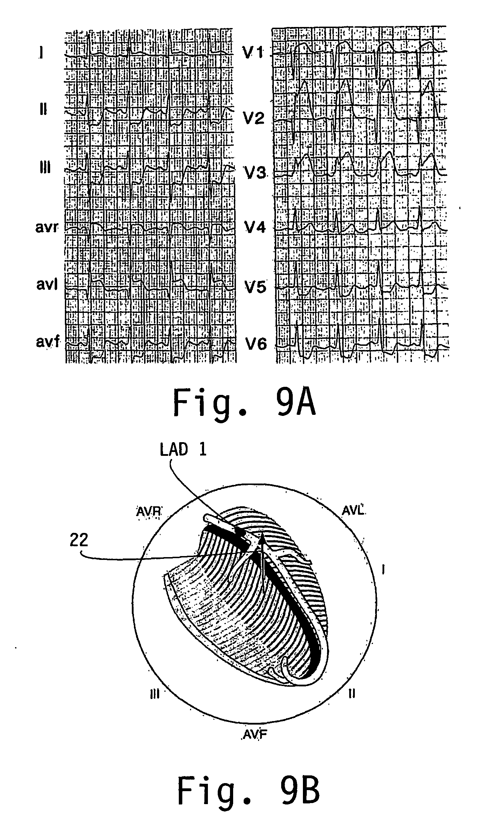

Of the twelve ECG signals that are shown in fig. 9A, the ECG signals II, III, AVF, V5 and V6 exhibit a depression of the ST segment ("-"), whilst the ECG signals I, AVR, AVL, V1, V2, V3 and V4 exhibit an elevation ("+") of the ST segment. None of the segments of the twelve ECG signals being measured exhibits or has the isoelectric level.

After analysis of the ST segment deviations of said the ECG signals, using the device according to the invention, an ST segment deviation vector 22 is composed, which points to the upper part (LAD 1) of the left-hand anterior descending coronary artery, as is shown in Fig. 9B. In Fig. 3B, the location of this occlusion is indicated at 4.

It can be concluded, also after analysis of the determined ST segment deviations of the various ECG signals by means of the diagnosis and analysis model that is shown in Fig. 6, that the measured deviations correspond most to the characteristic ST segment deviations associated with an LAD 1 occlusion.

In a similar manner, the ST segment deviations associated with each series of measured ECG signals can be correlated to an associated occlusion condition as defined in the diagnosis and analysis model of Fig. 6. With each type of occlusion, certain specific ECG signals exhibit a characteristic ST segment deviation (elevation or depression), whilst the ST segment associated with other measured ECG signals remains isoelectric or may exhibit an elevation or a depression.

The diagnosis and analysis model according to the invention can also be used for diagnosing the extent of coronary artery strictures in the case of unstable angina pectoris.

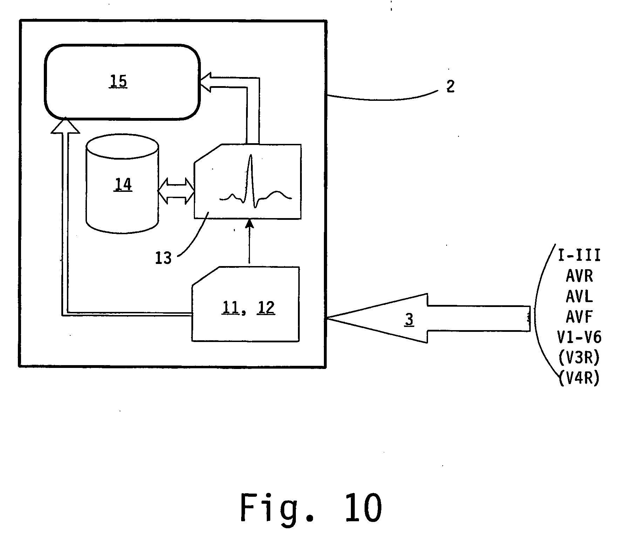

Fig. 10 schematically shows a further embodiment of a device according to the invention. The device is preferably a portable unit 2 comprising a bundle of ECG electrodes 3 (I-III, AVR, AVL, V1-V6 and optionally V3R and V4R), which can be placed on a person's body (see Fig. 2).

The bundle of ECG electrodes 3 is connected to the device 2 via an input connector (not shown), and the electrocardiograms of the heart as measured from various angles of incidence are read by the ECG data processing unit 11, 12 via said connector. In said processing unit 11, 12, not only the deprived ECG signals AVL, AVR and AVF are generated, but it is also determined in a known manner, on the basis of the ECG signals, whether an ischemic area is present in the patient's heart. The results of this known diagnostic technique can be presented on a display unit 15, for example a display screen, and be copied out on paper.

According to the invention, the device 2 comprises an ECG analysis unit 13. The analysis unit 13 samples the electric voltage signal of each electrocardiogram and determines the ST segment deviation from the isoelectric level. For every ECG signal that is measured (12 or 14), said ST segment sampling results in a differential voltage, which is positive in the case of an elevation and negative in the case of a depression.

The analysis unit 13 furthermore comprises a diagnosis unit 14. The twelve (or fourteen) sampled differential voltages corresponding to the ST segment deviations of the 12 (or 14) ECG signals I-III, V1-V6, AVF, AVL and AVR (and optionally V3R and V4R) are presented to the diagnosis unit 14. The diagnosis unit 14 functions in accordance with the diagnosis and analysis model of Fig. 6, i.e. the unit 14 is arranged to couple the series of sampled differential voltages to a more or less corresponding, previously defined occlusion condition present in the model.

Said coupling or "matching" can take place in a software-controlled manner. In that case the diagnosis unit 14 will contain storage means, memory means and a central processing unit, as well as a control system to be carried out by the central processing unit. The storage means may have a data processing programme stored therein, which programme, once it is loaded in the memory, is arranged for reading the digitally sampled differential voltages and which analyses the series of measuring values thus obtained and subsequently carries out an algorithm as described in Fig. 6 for coupling the series of analysed measuring values to a previously defined and stored occlusion condition.

In another embodiment, the diagnosis unit may be built up of electronic components that are known per se, which function as logic operators. In this way the diagnosis and analysis model of Fig. 6 can be configured as a logic circuit by means of logic operators. The presentation of the measured ST segment deviations to the logic circuit results in a coupling of the series of sampled ST segment deviations to a previously defined occlusion condition.

Said coupling is displayed via the display unit 15. Said displaying may take place textually or visually, in which former case the location of the occlusion in the coronary artery as well as the ST segment deviation vector are displayed. In the latter situation, the composed ST segment deviation vector is projected on a schematic representation of the heart. In that case the image being displayed concerns the location and the size of the ischemic area (occlusion) and the location of the occlusion in the coronary artery system of the heart, as well as the size of the ischemic area.

After feedback of the diagnosis of the ST segment deviations from the diagnosis unit 14 to the analysis unit 13, a suggestion for a treatment scheme may furthermore be made and be presented to the user (ambulance or hospital personnel and general practitioners) via the display unit 15. To that end, a relational database may be stored in the analysis unit 13, from which various treatment steps can be proposed in dependence on the occlusion that has been diagnosed. Think for example of referral to a hospital specialised in opening coronary arteries, administration of a clot-dissolving agent or transportation to a hospital for observation.

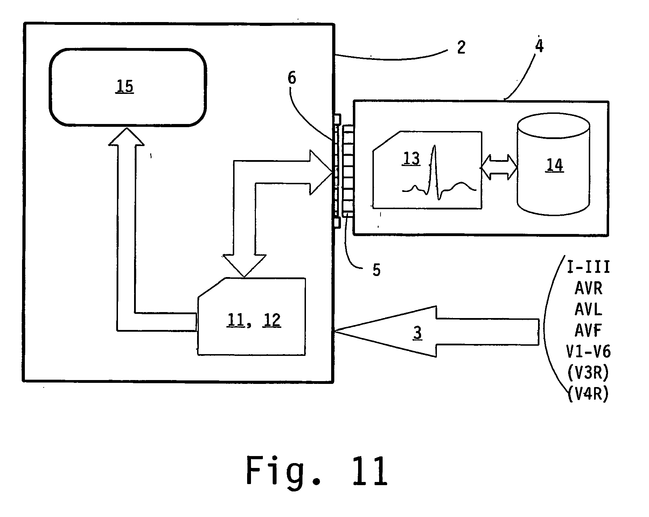

In another embodiment as schematically shown in Fig. 11, the device according to the invention is only built up of the ECG analysis unit 13 and the diagnosis unit 14, as shown in the embodiment of Fig. 10. This simplified unit, which may or may not be provided with an independent power source (battery), may function as an extension of already existing devices by means of which electrocardiograms can be made with human beings or animals.

This unit can be coupled to the existing device in a simple manner via a known cable connection. Likewise, the unit 13-14 may be provided with a connector 5, which makes it possible to click the unit to a corresponding connector 6 of the known device 2, so that an integrated ECG measuring and analysing device is obtained. This enhances the ease of use and makes the combined device more easily manageable, compact and portable.

The ECG signals V1-V6, I-III, AVF, AVL and AVR (and V3R and V4R, if necessary) being read by the processing unit 11,12 or 14 are passed on to the separate unit 4 via the connectors 5-6. In said separate unit 4, the ECG analysis and diagnosis steps are carried out in accordance with the algorithm that is described in Fig. 6, and the results are carried back to the processing unit 11, 12 and the display unit 15.

This simplified embodiment of the device according to the invention enables a low-cost upgrade of the existing ECG measuring apparatus to a device according to the invention.

## Claims

1. A device (2) for determining the presence of an ischemic area in a patient's heart, comprising:
a group of ECG electrodes (3) for measuring ECG signals from the heart of a patient;
ECG data processing means to be connected to the ECG electrodes, which are arranged for receiving the ECG signals and determining the presence of the ischemic area in the heart on the basis of said ECG signals; as well as
display means for displaying ECG data generated by the ECG data processing means to a user; **characterized in that** the device further comprises
ECG analysis means, which means are arranged for analysing the ECG signals (13) and determining the location of the occlusion in the coronary artery system of the heart (14), and for determining the iso-potential level (0) as well as the deviation of the ST segment from the iso-potential level for every EGG signal, and for composing an ST segment deviation vector on the basis of the ST segment deviations as determined (132),
said ECG analysis means further employing a diagnosis and analysis model, said model comprising a number of respective columns arranged side by side, each respective column individually containing values "+" , "0" or "-" of characteristic ST segment deviations for each of the ECG signals corresponding to a respective one of eight occlusions that are most likely to occur in the coronary artery system,
and furthermore said ECG analysis means are arranged for determining the location of the occlusion in the coronary artery system of the heart (14) by, after analysis of the determined ST segment deviations of the various ECG signals by means of said model, diagnosing the patient's occlusion based on concluding that the measured ST segment deviations correspond most to the characteristic ST segment deviations associated with said patient's occlusion.

2. A device according to claim, **characterized in that** the display means are arranged for displaying textual information and possibly also for projecting the ST segment deviation vector as composed on a schematic representation of the heart.

3. A device according to any one or more of the claims 1-2, **characterized in that** said diagnosis and analysis model can be used for diagnosing the extent of corronary artery strictures in the case of unstable angina pectoris.

## Patentansprüche

1. Vorrichtung (2) zum Feststellen des Vorliegens eines ischämischen Bereichs in einem Patientenherz, umfassend:
eine Gruppe von EKG-Elektroden (3) zum Messen von EKG-Signalen aus dem Herzen eines Patienten;
mit den EKG-Elektroden zu verbindende EKG-Datenverarbeitungsmittel, die dafür ausgelegt sind, die EKG-Signale zu empfangen und das Vorliegen des ischämischen Bereichs im Herz auf der Basis der EKG-Signale festzustellen; wie auch
Anzeigemittel, um durch die EKG-Datenverarbeitungsmittel erzeugte EKG-Daten einem Benutzer anzuzeigen; **dadurch gekennzeichnet, dass** die Vorrichtung weiter umfasst
EKG-Analysemittel, wobei die Mittel zur Analyse der EKG-Signale (13) und zum Feststellen des Orts des Verschlusses im koronaren Arteriensystem des Herzens (14), und zum Feststellen des Isopotential-Pegels (0) wie auch der Abweichung des ST-Segments aus dem Isopotential-Pegel für jedes EKG-Signal, und zum Zusammensetzen eines ST-Segment-Abweichungsvektors auf Basis der festgestellten (132)ST-Segment Abweichungen, ausgelegt sind,
wobei die EKG-Analysemittel weiterhin ein Diagnose- und Analysemodell einsetzen, das eine Anzahl entsprechender, Seite an Seite angeordneter Spalten umfasst, die alle individuell Werte "+", "0" oder "-" charakteristischer ST-Segment-Abweichungen für jedes der EKG-Signale entsprechend einem jeweiligen der achte Verschlüsse, die am wahrscheinlichsten im koronaren Arteriensystem auftreten, enthalten,
und weiterhin die EKG-Analysemittel zum Feststellen des Orts des Verschlusses im koronaren Arteriensystem des Herzens (14) ausgelegt sind, durch Diagnose, nach Analyse der festgestellten ST-Segment-Abweichungen der verschiedenen EKG-Signale mittels des Modells, der Patientenverschlüsse, basierend auf dem Schlussfolgern, dass die gemessenen ST-Segment-Abweichungen am meisten mit den charakteristischen ST-Segment-Abweichungen korrespondieren, die mit dem Patientenverschluss assoziiert sind.

2. Vorrichtung gemäß Anspruch 1, **dadurch gekennzeichnet, dass** das Anzeigemittel zum Anzeigen von Textinformationen und möglicherweise auch zum Projizieren des ST-Segment-Abweichungsvektors, wie zusammengesetzt auf einer schematischen Repräsentation des Herzens, ausgelegt ist.

3. Vorrichtung gemäß einem oder mehreren der Ansprüche 1 - 2, **dadurch gekennzeichnet, dass** das Diagnose- und Analysemodell zur Diagnose des Ausmaßes von koronaren Arterienstenosen im Falle instabiler Angina pectoris verwendet werden kann.

## Revendications

1. Dispositif (2) permettant de déterminer la présence d'une zone ischémique dans le coeur d'un patient, comprenant :
un groupe d'électrodes d'ECG (3) destiné à mesurer des signaux d'ECG provenant du coeur d'un patient ;
des moyens de traitement des données d'ECG à relier aux électrodes d'ECG, qui sont disposées pour recevoir les signaux d'ECG et déterminer la présence de la zone ischémique dans le coeur sur la base desdits signaux d'ECG ; ainsi que
des moyens d'affichage pour afficher des données d'ECG générées par les moyens de traitement des données d'ECG à un utilisateur ; **caractérisé en ce que** le dispositif comporte en outre
des moyens d'analyse d'ECG, lesquels moyens sont disposés pour analyser les signaux d'ECG (13) et pour déterminer l'emplacement de l'occlusion dans le système coronarien du coeur (14), et pour déterminer le niveau iso-potentiel (0) ainsi que l'écart du segment ST par rapport au niveau iso-potentiel pour chaque signal d'ECG, et pour composer un vecteur d'écart du segment ST sur la base des écarts de segment ST, comme déterminé (132),
lesdits moyens d'analyse d'ECG utilisant en outre un modèle de diagnostic et de d'analyse, ledit modèle comprenant un certain nombre de colonnes respectives disposées côte à côte, chaque colonne respective contenant individuellement des valeurs « + », « 0 » ou « - », des écarts caractéristiques du segment ST pour chacun des signaux d'ECG correspondant à une occlusion respective parmi huit occlusions qui sont le plus susceptibles de se produire dans le système coronarien,
et en outre lesdits moyens d'analyse d'ECG sont disposés pour déterminer l'emplacement de l'occlusion dans le système coronarien du coeur (14) en diagnostiquant l'occlusion du patient, après l'analyse des écarts déterminés du segment ST par rapport aux divers signaux d'ECG au moyen dudit modèle, en se basant sur la conclusion que les écarts mesurés du segment St correspondent plus aux écarts caractéristiques de segment ST associés à l'occlusion dudit patient.

2. Dispositif selon la revendication 1, **caractérisé en ce que** les moyens d'affichage sont disposés pour afficher des informations textuelles et probablement aussi pour projeter le vecteur d'écart du segment ST tel que composé sur une représentation schématique du coeur.

3. Dispositif selon l'une quelconque des revendications 1 à 2, **caractérisé en ce que** ledit modèle de diagnostic et d'analyse peut être utilisé pour diagnostiquer la portée des sténoses coronariennes dans le cas d'une angine de poitrine instable.
